# EUROPEAN PATENT APPLICATION

(11) **EP 0 559 307 A1**
(43) Date of publication of application: **08.09.1993**
(21) Application number: 93250072.1
(22) Date of filing: 04.03.1993
(51) Int. Cl.: C12N 5/06, C12N 5/10, A01K 67/02

(54) **Maturation procedure of the recipient ocytes for multiplying bovine embryos**

(30) Priority: 04.03.1992 US 846187; 12.02.1993 US 16636
(71) Applicant: ABS Global, Inc., New York, N.Y. 10169 (US)
(72) Inventor: Stice, Steven L., DeForest, Wisconsin 53532 (US)
(74) Representative: UEXKÜLL & STOLBERG

(57) **Abstract**

An improvement in the process of bovine multiplication by nuclear transfer is based on activation of the recipient oocytes. Activation is achieved by administering a shock to the oocytes, such as a temperature, biological, chemical or electric shock. After culture of oocytes at room temperatures, the recipient oocytes seem more amenable to nuclear transfer and higher percentages of viable nuclear transfer embryos can be achieved than from similar unactivated cultures.

## Description

This application is a continuation-in-part of Serial No. 07/846,187, filed March 4, 1992, pending in the United States Patent and Trademark Office.

### Field of the Invention

The present invention is generally directed to a process for multiplying bovine embryos, and is specifically directed to an improved process for maturation of the recipient oocyte so that the overall efficiency of the bovine embryo multiplication process is improved.

### Background of the Invention

Embryo multiplication by nuclear transfer involves the transplantation of the living nuclei from embryonic cells into recipient cells, typically unfertilized eggs. Such transfers are made in order to increase the number of genetically identical embryos which can be obtained from a donor embryo. Once a fertilized embryo has reached a cleavage stage of having at least two cells, it becomes practical to transfer the nuclei from such cells, or the entire cells themselves, into recipient oocytes which have been enucleated, to thereby create multiple genetically identical embryos from such fusions. By allowing each of the fused nuclear transfer embryos to develop to a multiple cell stage, and then repeating the nuclear transplantation procedure, large numbers of genetically identical nuclear transfer embryos can be created from an original donor embryo.

A limitation on the commercial use of this process as practiced to date arises from the fact that there are certain inefficiencies in the process as currently practiced. Not all of the nuclear fusions created result in viable embryos. Not all viable embryos created by nuclear fusion turn out to be capable of creating a viable pregnancy in the cow resulting in a live calf. Accordingly, effort is currently being directed toward optimizing the efficiencies at each step in the procedure, so as to make the overall procedure more economically practical.

The techniques of bovine nuclear transplantation are generally described in two U.S patents, U.S. Patent No. 4,994,384 and Patent No. 5,057,420, both of which describe procedures for the serial multiplication of bovine embryos. In the techniques described in each of those patents, oocytes are recovered from the ovaries or reproductive tract of cows. The oocytes are selected for proper stage, and then are enucleated by physical aspiration through a transfer pipette, leaving an enucleated oocyte which still retains its external membranes. Synchronously, a donor embryo of the proper cell staging, typically a cleavage or morulae stage, is manipulated so that one or more cells or blastomeres are removed from the embryo. The donor cell which, of course, includes its nucleus, is then inserted into the perivitelline space of the recipient oocyte. An electrical pulse is then applied to fuse the membranes of the donor cell and the recipient oocyte to thus create an activated, fused single cell embryo. That single cell nuclear transfer embryo can then be cultured either in vitro, or in the oviduct of a mammal, until a stage in which it can be implanted into a recipient cow. A significant number of the fused embryos will retain viability, can be transplanted surgically or non-surgically into the uteri of cattle, and will result in a live birth of genetically identical calves. In a mammal in vivo, ovaries and the embryonic cells contained within them are maintained at physiological (around 39°C) temperatures. Heretofore, it has been the custom to maintain the excised ovaries and embryos within them at physiological temperatures throughout the nuclear transplantation process, on the theory that such temperatures more closely approximate physiological conditions naturally encountered by the embryos, and thus more closely mimic natural conditions.

### Summary of the Invention

The present invention is summarized in that a process for bovine nuclear transplantation makes use of recipient oocytes that have been activated before nuclear fusion. Activation results in a higher level of viability of fused embryos created by the process.

In a preferred embodiment, recipient oocytes may be activated by a period of cold culture prior to transplantation of the donor nucleus or cell. Such a cold culture can be conducted at room temperatures (less than 30°C), which is cold only relative to physiological temperatures at which such cultures are normally conducted. In a second embodiment, natural oocyte fertilization by sperm followed by enucleation and nuclear transfer also activates recipient oocytes and increases fusion embryo viability. Other pre-fusion oocyte activation methods are also envisioned.

It is an object of the present invention to further develop and define a step in a bovine nuclear transplantation process so as to increase the overall efficiency of the process.

It is a feature of the present invention in that it is relatively easy to perform, since it requires that recipient embryos be cultured at room temperatures or in the presence of an activating agent, such culture conditions being relatively easy to maintain.

It is an objective of the present invention to aid in the overall efficiency of bovine transplantation and multiplication processes, so as to result in greater numbers of viable pregnancies and multiplied genetically identical calves.

Other objects, advantages, and features of the present invention will become apparent from the following specification.

### Description of the Invention

In accordance with the present invention, bovine oocytes destined to be the recipient of a nuclear transfer procedure are subjected to an activation step prior to nuclear transplantation procedure. When an activated oocyte is utilized for a nuclear transplantation procedure, the resulting nuclear transfer embryo will be found to be significantly more viable, and more likely to result in a viable pregnancy and a calf, than comparable embryos resulting from oocytes which have not been subjected to an activation procedure.

Activation is most preferably done by culturing the recipient oocyte at a reduced sub-physiological temperature, in essence applying a cold, or actually cool, temperature shock to the oocyte. This may be most conveniently done by culturing the oocyte at room temperatures, which is cold relative to the physiological temperature conditions to which oocytes are normally exposed. This is the most preferred activation protocol because it involves no apparatus and no additions to the culture medium. While cold culture shock activation is a convenient way to practice the invention, the invention is not limited to activation by cold culture. Rather, activation may be achieved, for example, using a host of activation agents. Penetration of the oocyte by sperm during fertilization is also herein shown to activate prefusion oocytes which yield greater numbers of viable pregnancies and multiplied genetically identical calves after nuclear transfer. Other treatments, such as electrical shock and chemical shock, may also activate recipient oocytes for nuclear fusion.

The activated oocytes for use within the present invention are intended for a protocol of bovine embryo multiplication through nuclear transplant. It is appropriate therefore, at this juncture, to briefly describe the overall bovine embryo multiplication procedure. Bovine ovaries are collected at the slaughterhouse, and maintained in physiological saline for transportation from the slaughterhouse to the laboratory. Follicles ranging in size from 2 to 10 mm in diameter are then aspirated from the bovine ovaries. The immature oocytes contained within the follicular fluid are removed, HEPES buffered, and washed in hamster embryo culture medium (HECM) (described in Seshagire et al., Biol. Reprod., 40, pp. 599-606 (1989), and then placed into drops of maturation medium consisting of 50 microliters of tissue culture medium (TCM) 199 containing 10% fetal calf serum with appropriate gonadotropins luteinizing hormone (LH) and/or follicle stimulating hormone (FSH), and estradiol under a layer of lightweight paraffin or silicon at 39°C. After twenty hours in the maturation media, the oocytes are removed and placed in HECM containing 1 milligram per milliliter of hyaluronidase. The cumulus cells are removed from the oocytes by repeated pipetting through very fine bore pipettes. The stripped oocytes are screened for polar bodies, and the selected metaphase II oocytes, as determined by the presence of the polar bodies, are then used further in the transplantation and multiplication procedures.

The oocyte enucleation portion of the cloning procedure is described in U.S. Patent No. 4,994,384, the specification of which is hereby incorporated by reference. Briefly, the metaphase II oocytes are either placed in HECM containing 7.5 micrograms per milliliter cytochalasin B for immediate enucleation or are placed in CR1aa (described below), plus 10% estrus cow serum, and are enucleated later, preferably sixteen to eighteen hours later. Enucleation is accomplished using a micropipette to remove the polar body and the surrounding cytoplasm. The oocyte can then be screened to determine which oocytes have been successfully enucleated. This screening is successfully done by staining the oocytes with 1 microgram per milliliter 33342 Hoechst dye in HECM, and then viewing the oocytes under ultraviolet irradiation for less than 10 seconds. The oocytes that have been successfully enucleated can then be placed in culture media, preferably CR1aa plus 10% estrus cow serum.

After enucleation, a blastomere from a preimplantation donor embryo is placed in the perivitelline space, by the method also described in the above-identified Patent No. 4,994,384. For in vitro matured oocytes, this process is normally performed between 36 and 46 hours after the oocyte was first placed in the maturation media. After the blastomere cell is transferred into the perivitelline space of the recipient oocyte, the two cells are fused together by electrofusion. The cells to be fused are placed between two electrodes which are 500 microns apart, referred to as the fusion chamber, which contain Zimmerman's fusion medium. Up until the point of fusion, in previous techniques, the donor cells and the recipient cells have been maintained at 39°C, a physiological temperature. After fusion, the nuclear transfer embryos thus created are then placed in CR1aa plus 10% estrus cow serum at 39°C for 5 to 9 days. A detailed description of a process for culturing in vitro such nuclear transfer embryos can be found in published PCT patent application WO 90/13627. At the end of this culture period, the embryos can be judged for developmental rates. The embryos judged to have developed normally, either into morulae or blastocysts, can be transferred into recipient animals, and an offspring may be obtained after a normal pregnancy.

The above brief description of the embryo multiplication and nuclear transplant process has been altered in the inventive method described herein only prior to the cell fusion step. It has been found that activation of the oocytes during the maturation period increases developmental rates in the resulting nuclear transfer embryos. The oocytes may be cultured in CR1aa medium plus 10% estrus cow serum, just as has been done with the above procedure absent activation.

Cold culture of the oocytes may be done at any temperature significantly below physiological temperatures (below 30°C) and above freezing, preferably in the range of 24°C to 26°C. Even a brief period of cold culture (approx. 2 hours) just prior to fusion has proven useful in increasing the viability of the nuclear transfer embryo. A period of cold culture extending over a longer period (20-24 hours) has proven at least equally effective in increasing the survival and viability of the nuclear transfer embryos. The period of cold culture activation can be continuous, but it has also been found that two separated cold culture episodes, at the beginning and end of the in vitro oocyte maturation process, also increases survival and viability.

CR1aa embryo culture medium is described in the following Table 1.

**TABLE 1**

| CR1AA EMBRYO CULTURE MEDIUM | |
|---|---|
| Component | Concentration |
| NaCl | 114 mM |
| KCl | 3.1 mM |
| NaHCO3 | 25 mM |
| Sodium pyruvate | 0.4 mM |
| Hemicalcium lactate | 5 mM |
| Glutamine | 1 mM |
| MEM amino acids | 1 ml/100ml |
| BME amino acids | 2 ml/100ml |
| Gentamicin | 50 µg/ml |
| Estrus Cow Serum (ECS) | 10% |

Oocytes may also be activated by sperm during fertilization in vitro by culture in fertilization medium containing sperm for a period of time sufficient to allow the sperm to penetrate the oocytes. Preferably, the oocytes are fertilized by exposure to sperm at 20 hours after metaphase II selection, and the pronuclei are removed at 39 hours. The pronuclei of recently fertilized oocytes may be revealed using Hoechst dye 33342. Preferably, only those activated oocytes having two pronuclei are enucleated and used as recipient oocytes in the nuclear transfer process described above.

Other activation techniques may he used as well, including electric shock and chemical treatment. What is new to the process here is the performance of activation separate and prior to the step of inserting the donor nucleus. This prior activation enhances the overall efficiency of the process.

The examples below demonstrate the utility of an activation step and the protocol-independent nature of the activation step. As set forth below, the increase in development after nuclear transfer into activated oocytes can readily be ascertained by parallel experiments in which oocytes are prepared with or without a pre-fusion activation step. Furthermore, the efficacy of various activation steps may be compared by parallel activation steps followed by nuclear transfer.

### Examples

### Cold Culture Activation

In the cold culture examples described here, the embryo multiplication procedure described above was repeated with multiple groups of oocytes which had been cultured in differing conditions as described below. In all of these examples, the oocytes removed from the follicles were matured in vitro first for 20-24 hours prior to selection of the oocytes for metaphase II staging. The selected metaphase II oocytes were then cultured (or matured) for an additional 14-24 hour period either at physiological temperatures (about 39°C) or at room temperature (23-26°C), or combinations thereof. All nuclear transfers were performed using entire blastomere cells. The nuclear transfer embryos were either cultured in vitro or in ligated sheep oviducts prior to screening for morula or blastocyst development.

Shown in Table 2 below are the summarized results of a series of replicates in which either or both of the donor blastomeres and the recipient oocytes were cultured at physiological temperatures (designated 39C) or under cold culture of 23-26°C (designated CS) . These results demonstrate an increased rate of nuclear transfer embryo development for a cold culture oocyte, but no effect for a cold cultured donor cell.

**TABLE 2**

| Treatment | No. Recovered From Culture | No. Developed to Morulae or Blastocyst | Percent Developed |
|---|---|---|---|
| 39C oocyte and 39C donor cell | 199 | 9 | 5% |
| 39C oocyte and CS donor cell | 169 | 3 | 2% |
| CS oocyte and CS donor cell | 237 | 42 | 18% |

To verify that this statistically significant result was principally due to the cold culture of the recipient oocyte rather than the donor cell, the experiment was replicated using all four possible temperature permutations, and the data is summarized in the following Table 3.

**TABLE 3**

| Treatment | No. Recovered From Culture | No. Developed to Morulae or Blastocyst | Percent Developed |
|---|---|---|---|
| 39C oocyte and 39C donor cell | 80 | 1 | 1% |
| 39C oocyte and CS donor cell | 131 | 8 | 7% |
| CS oocyte and 39C donor cell | 44 | 5 | 11% |
| CS oocyte and CS donor cell | 134 | 19 | 14% |

A comparison of the first and third conditions above indicates clearly that the room temperature culture of the recipient oocytes is the biggest factor in efficiency improvement. The difference between conditions three and four is not considered significant.

Several replicates were conducted to compare the timing of the cold culture period. A cold culture period of 20-22 hours after metaphase II selection and prior to transfer and fusion was the best of the conditions tested. A cold culture period limited to two hours after the selection of metaphase II oocytes proved of limited improvement, but a regimen of two hours of cold culture (25°C) followed by sixteen hours of physiological culture (39°C) and two additional hours of cold culture (25°C) was of greater improvement. Slight timing change, such as 3 hours rather than 2 hour cold culture, or 22 hours as compared to 20 hours, seemed to make little significant difference.

These results demonstrate a clearly discernible trend toward increased nuclear transfer embryo viability and survival when the recipient oocyte has received a cold culture treatment for a time period in excess of 2 hours and within 24-48 hours prior to cell fusion. This cold culture is believed to serve as a form of pre-fusion activation of the recipient oocyte. Whatever the cause of the phenomenon, it can be used to increase the efficiency of a practical bovine embryo multiplication protocol.

### Fertilization activation

In this example, nuclear transfer into enucleated oocytes after in vitro activation by natural oocyte fertilization was compared against nuclear transfer after cold culture activation. One population of pre-fusion oocytes were activated by cold culture at 23-27 °C for three hours before enucleation, while a second group of oocytes were activated by sperm penetration according to the following protocol. At 20 hours after metaphase II selection, the second group of oocytes were placed in fertilization medium and were exposed to sperm for approximately 18 hours, at which time the fertilized oocytes contained pronuclei that were roughly 25 µm in diameter. The fertilized oocytes were enucleated with the aid of Hoechst dye 33342. Only oocytes which had two pronuclei and which could be enucleated were used as recipient oocytes.

Both sets of enucleated oocytes were used as recipients in the nuclear transfer protocol described above. As shown in Table 4, sixteen percent of the nuclear transfer embryos derived from fertilized oocytes matured to the morula and blastocyst stage. This rate did not differ significantly from the twenty two percent development rate obtained using cold cultured oocytes.

The data of this example demonstrate that activation of a recipient oocyte may be achieved using natural sperm fertilization and with cold culture. As such, activation before fusion appears to be a generally advantageous step in the preparation of recipient oocytes in a nuclear transfer protocol, without regard to the activation method chosen.

**TABLE 4**

| Activation protocol | No. of Nuclear Transfers | Development to Morula and Blastocyst (Percent Development) |
|---|---|---|
| Cold Culture | 112 | 25 (22%) |
| Fertilization | 80 | 13 (16%) |

## Claims

1. A method of producing a multiplied bovine embryo comprising the steps of
(a) isolating a recipient oocyte;
(b) enucleating the recipient oocyte;
(c) activating the recipient oocyte;
(d) isolating a donor blastomere cell from a donor embryo;
(e) introducing the donor blastomere cell into the perivitelline space of the recipient oocyte;
(f) fusing the donor blastomere cell into the recipient oocyte to make a nuclear transfer embryo; and
(g) transferring the nuclear transfer embryo into a maternal animal.

2. A method as claimed in claim 1 wherein after the isolating step (a), the oocyte is matured in the presence of gonadotrophins prior to the activation step.

3. A method as claimed in claim 2 wherein after the isolating step (a) and the maturing in the presence of gonadotrophins, the oocytes are selected for metaphase II condition.

4. A method as claimed in claims 1 to 3 wherein the activating step (c) is by cold culture for a time period in excess of 2 hours and at a temperature of less than 30°C.

5. A method as claimed in claim 4 wherein the activating step (c) is conducted at a room temperature of between 23° and 26°C.

6. A method as claimed in claims 1 to 5 wherein the activating step (c) is conducted for a time period of at least 20 hours.

7. A method as claimed in claims 1 to 6 wherein the activating step (c) is by fertilizatin in the presence of sperm.

8. A method as claimed in claims 1 to 7 wherein the activating step (c) is conducted for a time period of about 18 hours.

9. A method of preparing recipient bovine oocytes for a nuclear transfer process according to claims 1 to 8 comprising the steps of
(a) recovering ovaries from cows;
(b) isolating immature oocytes from the ovaries;
(c) maturing the ovaries in the presence of gonadotrophins at a physiological temperature;
(d) selecting among the matured oocytes for metaphase II oocytes;
(e) enucleating the activated oocytes; and
(f) activating the selected oocytes.

10. A method as claimed in claim 9 wherein the maturation step (c) is conducted for 20-24 hours.

11. A method as claimed in claims 9 or 10 wherein the selection step (d) is performed by examining for the presence of polar bodies.

12. A method as claimed in claims 9 to 11 wherein the activating step (f) is by a cold culture for a time period in excess of 2 hours and at a temperature less than 30°C.

13. A method as claimed in claim 12 wherein the activating step (f) is conducted at a room temperature of between 23° and 26°C.

14. A method as claimed in claims 9 to 13 wherein the activating step (f) is conducted for a time period of at least 20 hours.

15. A method as claimed in claims 9 to 14 wherein the activating step (f) is by fertilization in the presence of sperm.

16. A method as claimed in claims 9 to 15 wherein the activating step (f) is conducted for a time period of about 18 hours.
